# EUROPÄISCHE PATENTSCHRIFT

(11) **EP 2 744 504 B1**
(45) Veröffentlichungstag und Bekanntmachung des Hinweises auf die Patenterteilung: **10.06.2015**
(21) Anmeldenummer: 12762228.0
(22) Anmeldetag: 20.08.2012
(51) Int. Cl.: A61K 36/185, A61K 36/515, A61K 36/70, A61K 36/85, A61P 11/12, A61P 31/00

(54) **VERFAHREN ZUR HERSTELLUNG VON TROCKENEXTRAKTEN**
METHOD FOR PRODUCING DRY EXTRACTS
PROCÉDÉ DE FABRICATION D'EXTRAITS SECS

(30) Priorität: 19.08.2011 EP 11178206; 15.12.2011 EP 11193734; 30.05.2012 EP 12170125
(43) Veröffentlichungstag der Anmeldung: 25.06.2014
(73) Patentinhaber: Bionorica SE, 92318 Neumarkt (DE)
(72) Erfinder: POPP, Michael, 91207 Lauf-Heuchling (DE)
(74) Vertreter: Simandi, Claus
(86) Internationale Anmeldenummer: PCT/EP2012/066212
(87) Internationale Veröffentlichungsnummer: WO 2013/026830

(56) Entgegenhaltungen:
- DE-B3-102005 053 926

## Beschreibung

Die Erfindung betrifft ein Verfahren zur Herstellung von Pflanzentrockenextrakten und diese enthaltenden pharmazeutischen Zubereitungen, insbesondere Phytopharmaka, welche wenigstens einen ethanolischen/wässrigen Extrakt aus einer Pflanzen(droge) enthält, wobei die Pflanzen ausgewählt sind aus der Gruppe bestehend aus: *Rumex acetosa L., Rumex acetosella L., Rumex obtusifolius L., Rumex patientia L.,* und *Rumex crispus L.,* (im Folgenden mit dem Sammelbegriff *"Rumicis herba"* bezeichnet); *Verbena officinalis; Sambucus nigra; Primula veris;* und *Gentiana lutea* sowie deren Mischungen. Die Erfindung betrifft ferner ein Arzneimittel zur Behandlung von entzündlichen und/oder infektiösen Erkrankungen des Nasen-Rachen-Raums und/oder der Nasen-Nebenhöhlen als auch ein Nahrungsergänzungsmittel sowie deren Verwendung.

Die obigen Arzneipflanzen sind als Sekretolytika bei Infekten der oberen Atemwege, insbesondere bei Sinusitiden bekannt. Jede Einzeldroge trägt dabei einen Anteil zur einzigartigen Wirksamkeit der Zusammensetzung bei:
Von *Gentiana lutea* (Gelber Enzian, Gentian root, Enzianwurzel) wird in der Regel die Wurzel arzneilich verwendet. Unter den Inhaltsstoffen befinden sich unter anderem verschiedene Secoiridoidglykoside mit schleimlösender Wirkung.
Von den genannten *Rumex-*Arten, im Folgenden *"Rumicis herba"* ((Sauer)Ampferkraut, Sorrel herb) genannt, werden in der Regel Blätter und Stengel arzneilich verwendet. Hierin finden sich Flavonoide und verschiedene Gerbstoffe als Inhaltsstoffe mit entzündungshemmender Wirkung, welche zudem die körpereigenen Abwehrkräfte positiv unterstützen.

Unter dem Sammelbegriff "Rumicis herba (Sorrel herb)" wird eine Mischung folgender Spezies verstanden:
Rumex acetosa L., Synonym: Lapathum acetosa SCOP, Synonym: Lapathum pratense LAM, Synonym: Acetosa pratensis MILLER; Rumex acetosella L, Synonym: Rumex infestus SALISB;
Rumex obtusifolius L., Synonym Lapathum obtusifolium MOENCH, Synonym Lapathum obtusantum MONTAD, Synonym Rumex actus WALLR. Synonym Rumex silvestris WALLR;
Rumex patientia L., Synonym Rumex olympicus BOISS., Synonym Lapathum hortense MOENCH;
Rumex crispus L.;
Rumex thyrsiflorus FINGERH., Synonym Acetosa thyrsiflora FINGERH, Synonym Rumex acetosa subsp. auriculatus WALLR.

Von *Verbena officinalis* (Eisenkraut, Verbena herb) werden vorzugsweise Blätter und Stengel arzneilich verwendet, die als Hauptinhaltsstoffe Iridoidglykoside, Phenylethanoidglykoside und Flavonoide enthalten, wodurch schleimlösende und antivirale Wirkungen erzielt werden.

Von *Sambucus nigra* (Schwarzer Holunder, Elder flower, Holunderblüten) werden typischerweise die Blüten arzneilich verwendet, deren Inhaltsstoffe verschiedene Flavonolglykoside enthalten und als Hauptwirkstoff Sambunigrin, ein cyanogenes Glykosid enthält, welche schleimlösend und antiviral wirken (Grabovac, A. und Ullmer, A., Österreichische Apotheker-Verlagsgesellschaft m.b.H., 2003).

Von *Primula veris* (Schlüsselblume, Primula flower, Schlüsselblumenblüten) werden Blüten und Kelch arzneilich verwendet. Die Inhaltsstoffe umfassen Triterpensaponine sowie Phenolglykoside, wie Primulaverin. Sie wirken schleimlösend und bekämpfen Viren. Die Inhaltsstoffe wirken als mildes Sekretolytikum und Expektorans bei der Behandlung von Atemwegserkrankungen.

Die Kombination der genannten Heilpflanzen ist als Sekretolytikum unter der für die Anmelderin eingetragenen Marke Sinupret® bekannt und seit etwa 75 Jahren auf dem Markt. Die in Sinupret® verwendeten Heilpflanzen werden gezielt ausgewählt, untersucht und weiterverarbeitet. Die hierdurch erzielte gleich bleibende Qualität des Arzneimittels erreicht der Hersteller, die Firma BIONORICA, durch optimierte Anzuchtund Erntestrategien sowie einer strengen Qualitätskontrolle.

Die dem Sinupret® zugrunde liegende Zusammensetzung ist vorzugsweise bei Entzündungen sowie Infektionen des Hals-Nasen- und Ohrenbereiches wirksam und eignet sich insbesondere für die Behandlung der akuten und chronischen Sinusitis und / oder Rhinosinusitis.

Sowohl akute als auch chronische Sinusitiden treten häufig auf. In drei von vier Fällen entwickelt sich die Sinusitis als Folge eines Schnupfens mit einer Ausweitung auf die Nasennebenhöhlen, die von einer Schleimhautentzündung begleitet ist. Die Atemwege reichen von der Nasen-Haupthöhle mit den verschiedenen Nebenhöhlen bis zu den Lungenbläschen. Zu den Nasen-Nebenhöhlen zählen die Stirnhöhlen, die Siebbeinzellen, die Keilbeinhöhle und die Kieferhöhlen. Sämtliche genannten Knochenhöhlen sind innen mit Schleimhaut ausgekleidet und münden über enge Öffnungen, den Ostien, in die Nasenhaupthöhle.

Die Oberfläche der Atemwege ist mit einem schützenden Schleim überzogen, an dem Schmutzpartikel und Krankheitserreger wie z.B. Viren, Bakterien oder Pilze, die mit der Atemluft eindringen, haften bleiben. Der Schleim enthält Abwehrstoffe, die die eindringenden Stoffe angreifen und unschädlich machen. Damit die Fremdstoffe aus dem Körper ausgeschwemmt werden können, wird der Schleim in der Regel mit Hilfe der Flimmerhärchen des Flimmerepithels in Richtung Rachen abtransportiert, wo er verschluckt werden kann.
Um infektbedingte Atemwegserkrankungen abwehren zu können, muss die Schleimhaut ungehindert über Schutz- und Reinigungsmechanismen verfügen. Für den Abtransport des mit Erregern beladenen Schleims ist die ungehinderte Funktion der Flimmerhärchen unabdingbar, die durch wellenförmige Bewegungen den Schleim weitertransportieren. Bei einem Infekt sowie bei Entzündungsprozessen der oberen Atemwege sind die Schutz- und Reinigungsmechanismen der Schleimhaut eingeschränkt funktionsfähig.

Beispielsweise lösen Viren wie Rhinoviren, Adenoviren oder Coronaviren Entzündungsreaktionen der Schleimhäute aus, wodurch die Schleimhaut anschwillt und verstärkt Schleim produziert. Dabei kommt es zunächst zu wässrigem, anschließend zu zähflüssigem Schleimfluss. Im Laufe der Entzündung der Nasenschleimhaut können die Ostien der Nebenhöhlen anschwellen und den Abfluss des Schleims behindern oder sogar verhindern. Dies führt zu einem Stau in den Nebenhöhlen verbunden mit zähem Schleim, welches zu einer Funktionseinbuße bzw. Funktionsverlust der Flimmerhärchen führt. Dies bewirkt schließlich eine Einbuße des Reinigungsmechanismus der Schleimhaut.

Ein solches Mikroenvironment begünstigt die schnelle Vermehrung der ubiquitär vorhandenen Mikroorganismen.
Bei längerer Dauer dieser ungünstigen Bedingungen, wie z.B. eine geschwollene Schleimhaut und durch zähflüssigen Schleim verklebte Flimmerhärchen, kann es zu einer chronischen Sinusitis kommen, mit der Folge einer dauerhaften Schädigung der Schleimhaut und des Flimmerepithels. Atemwegsrelevante Erreger, worunter auch insbesondere HNO-relevante Keime verstanden werden, die sich im Schleim einnisten, sind beispielsweise Staphylococcus aureus, Staphylococcus epidermidis, Streptococcus pyogenes, Streptococcus pneumoniae, Streptococcus mutans oder Haemophilus influenzae.

Im Falle einer Verklebung der oberen Atemwege durch dickflüssigen Schleim induzieren die Inhaltsstoffe der verwendeten Zusammensetzung (Gentiana lutea : Rumicis herba : Verbena officinalis : Sambucus nigra : Primula veris = 1:3:3:3:3) die Bildung von frischem, dünnflüssigen Schleim, wodurch der oben beschriebene Prozess der Schleimlösung und des Abtransportes sowie Verminderung der Entzündungssymptome erzielt und Heilungsprozess der Nasenschleimhaut eingeleitet wird. Sinupret® bewirkt auf schonende Art und Weise die Wiederherstellung der Selbstreinigungskraft der Atemwege und entfaltet gleichzeitig eine starke antimikrobielle Wirkung. Ein Kennzeichen von Sinupret® ist seine gute Verträglichkeit, der von BIONORICA ermittelten Zusammensetzung und Dosierung, welche selten Nebenwirkungen beim Patienten hervorrufen und zudem sind keine Wechselwirkungen mit anderen Arzneimitteln bekannt.

Pflanzentrockenextrakte sind allgemein beschrieben und Pflanzentrockenextrakte sind aus wässrig/ethanolischen Auszügen bekannt.

Pflanzentrockenextrakte können beispielsweise gemäß der technischen Lehre von EP0753306 in großen Mengen hergestellt werden.

Es besteht jedoch ein großes Bedürfnis einen neuen Sinupret®-Pflanzentrockenextrakt bereitzustellen, der eine vorteilhafte verbesserte Wirkung der Pflanzenkombination aufweist.

Das Deutsche Arzneimittelbuch (DAB) 2010 setzt Mindestgehalte von Inhaltsstoffen für die Drogenqualität fest, so dass für eine konstante und verbesserte Qualität vermehrte Anstrengungen getroffen werden müssen. Gerade Extraktions- und Trockenverfahren stellen ein Bottleneck für eine ausreichende Qualität von Phytopharmaka dar.

Ausgehend vom diesem Stand der Technik ist es daher Aufgabe der vorliegenden Erfindung, ein verbessertes Verfahren zur Herstellung eines Pflanzentrockenextraktes bereitzustellen als auch einen verbesserten Pflanzentrockenextrakt als solchen der wenigstens einen ethanolischen/wässrigen Extraktionsschritt enthält, wobei die Pflanzen ausgewählt sind aus der Gruppe bestehend aus: *Rumex acetosa L., Rumex acetosella L., Rumex obtusifolius L., Rumex patientia L.,* und *Rumex crispus L.,* (im Folgenden und in den Ansprüchen mit dem Sammelbegriff *"Rumicis herba"* bezeichnet); *Verbena officinalis; Sambucus nigra; Primula veris;* und/oder *Gentiana lutea* sowie deren Mischungen.

Überraschender Weise konnte mittels einer zweifachen Extraktion, wobei in einem ersten Schritt eine wässrige/ethanolische Extraktion und in einem zweiten Schritt eine wäßrige Extraktion erfolgt, ein verbesserter Pflanzentrockenextrakt erhalten werden.

Überraschender Weise kann mittels diesem erfindungsgemäßen Verfahren der Gehalt an vorteilhaften Wirkstoffen im bestehenden Wirkstoffgemisch im jeweiligen Gesamtextrakt nach Trocknung angereichert werden, so dass eine verbesserte pharmakologische Wirksamkeit des erhaltenen Pflanzentrockenextraktes erreicht wird.

Daher betrifft die Erfindung ein Verfahren zur Herstellung von Pflanzentrockenextrakten, mit den Schritten:
a.) alkoholische/wässrige Extraktion von Rumicis herba, Verbena officinalis, Sambucus nigra, Primula veris und Gentiana lutea,
b.) Abtrennen des Überstandes,
c.) zweite wässrige Extraktion des Rückstandes aus a.),
d.) Abtrennen des Überstandes,
e.) Vereinen der erhaltenen Überstände aus b.) und d.),
f.) Trocknen der Überstände und Erhalten des Pflanzentrockenextraktes.

Die verfahrensgemäße Verbesserung besteht darin, dass gegenüber einem üblichen einfachen wässrig-ethanolischen Extrakt eine schnellere Wirksamkeit erreicht wird, siehe Beispiele. Dies ist überraschend, da ein Wechsel des Lösungsmittels zur Extraktion allenfalls eine lineare Extrapolation hätte erwartet werden können (z.B. falls ausschließlich wässrig/ethanolisch oder ausschließlich wässrig extrahiert wird).

Insbesondere erlaubt das erfindungsgemäße Verfahren eine verbesserte Dosierung, so dass bei gleicher Dosis ein erhöhter curativer Effekt erreicht werden kann.

Daher betrifft die Erfindung ebenfalls einen Pflanzentrockenextrakt, der nach einem erfindungsgemäßen Verfahren erhalten wird oder erhältlich ist (nachstehend erfindungsgemäßer (Pflanzen)Trockenextrakt, Sinupret Trockenextrakt (TE)).

In einer weiteren bevorzugten Ausführungsform des erfindungsgemäßen Verfahrens ist das Verhältnis von Gentiana lutea : Rumicis herba : Verbena officinalis : Sambucus nigra : Primula veris 1:3:3:3:3, jeweils +/- 0,3 bis 0,5 beträgt (wie z.B. 1 : 3,2 : 2,9 : 2,5 : 3,2 : 3).

Weiterhin ist bevorzugt, dass eine Pflanzengesamtheit aller Pflanzen(drogen) in einem Batch vorgelegt ist, wobei das Verhältnis der Pflanzen(drogen) wie vorstehend genannt ist. Weiterhin ist bevorzugt, dass die vorgelegten Pflanzen(drogen) gereinigt und geschnitten sind.

In einer weiteren bevorzugten Ausführungsform des erfindungsgemäßen Verfahrens, weist in Schritt a.) das wässrige/alkoholische Extraktionsmittel einen Gehalt 40 : 60 (v/v) bis 60 : 40 (v/v), insbesondere 41 : 59 (v/v) oder 50 : 50 (v/v) Wasser/Alkohol auf. Ethanol ist bevorzugt, ebenfalls umfasst sind jedoch Methanol und Propanol oder Mischungen davon. Weiterhin ist die Verwendung von 96 %-igen Ethanol bevorzugt.

Ebenfalls ist erfindungsgemäß vorgesehen, dass die Extraktion in den Schritten a.) und c.) bei 20 bis 40 DEG C durchgeführt wird, wobei die Extraktion in den Schritten a.) und c.) in 2 bis 8h durchgeführt wird.

Im Sinne dieser Erfindung kann ein "Abtrennen des Überstandes" nach erfindungsgemäßer Extraktion mittels Ablaufen, Dekantieren, Filtration, Sieben oder eines dem Fachmann bekannten Trennverfahren kontinuierlich oder diskontinuierlich erfolgen.

Weiterhin ist bevorzugt, dass das Trocknen gemäß Schritt f.) im Vakuum bei 30 bis 60 DEG C, insbesondere bei 40 bis 50 DEG C, vorzugsweise in einem Vakuumrührwerktrockner erfolgt.
Der erfindungsgemäße Trockenextrakt weist einen RestethanolGehalt von max. 0,5 % auf.

Weitere geeignete erfindungsgemäße Trockenverfahren werden erläutert:
Pflanzentrockenextrakte werden üblicherweise hergestellt, wobei ein Pflanzenmaterial mit Hilfe eines Lösungsmittels oder Lösungsmittelgemisches extrahiert, beispielsweise mittels Mazeration oder Perkolation und nach Abtrennung des Extraktionsrückstandes, den erhaltenen Fluidextrakt bzw. die erhaltene Tinktur zur Trockne einengt.

Herkömmliche Trocknungsverfahren sind erfindungsgemäß umfasst und schließen die Wirbelschichttrocknung oder die Einengung zu einem Dick- oder Spissumextrakt und anschließende Vakuumbandtrocknung oder Hordentrocknung dieses Spissumextraktes ein.

Ebenfalls können klassische Verfahren zur Herstellung von erfindungsgemäßen Pflanzentrockenextrakten über einen Fluidextrakt (bzw. flüssigen Pflanzenextrakt) oder eine Tinktur berücksichtigt werden; wobei nach anschließender Abdestillation der Lösungsmittel ein so genannter Spissumextrakt (zähflüssiger Extrakt) erhalten wird, dem häufig Hilfsstoffe und/oder Zusatzstoffe wie z.B. Lactose, Polyvinylpyrrolidon, Saccharose, Siliciumdioxid usw. zugesetzt werden. Diese feuchte, zähe Masse wird dann in Hordenschränken oder Trocknern zur gewünschten Trockenextraktzubereitung gebracht.

Ein Verfahren, das sehr häufig in der Trockenextraktherstellung benutzt wird, ist das sog. Vakuumbandtrocknungsverfahren. Hierbei wird der Spissumextrakt nach einer Vortrocknung über Fallstromverdampfer zur Trockenextraktzubereitung gebracht.

Ein Trocknungsverfahren mittels eines Wirbelschichttrockners benötigt Temperaturen zwischen ca. 47 DEG C und 117 DEG C. Die Trocknung in diesem Verfahren wird unter normalen Druckbedingungen durchgeführt.

Ein schonendes Trocknungsverfahren zur Gewinnung von erfindungsgemäßen Pflanzentrockenextrakten ist in der EP 0 753 306 beschrieben. Gemäß dem beschriebenen Verfahren wird der in der Extraktion gewonnene Fluidextrakt aus den Pflanzenmaterialien gemäß dem erfindungsgemäßen Verfahren in einem Vakuumtrocknungssystem, vorzugsweise Vakuumrührwerktrockner mit einem sich durch eine zylindrische Misch-und Trockenkammer erstreckenden mehrschenkligen Rührwerk mit eigenem Antrieb, sowie erforderlichenfalls versehen mit Brüdenfilter, Rückspülungseinrichtung, Lösungsmittelkondensator mit Nachkühler und Auffanggefäß, Rückkondensator und einer Prozess-, Steuer-und Regeleinheit, sowie gegebenenfalls mit Granulierdüsen, eingebracht und in der mit einem sich über die Gesamttiefe der Misch-und Trockenkammer erstreckenden Zerhacker mit durch einem kammförmigen Stator hindurch rotierenden Messern ausgestattetem Trockner bei einer Vor-und Rücklauftemperatur zwischen 120 DEG C und 5 DEG C, einer Innenraumtemperatur zwischen 10 DEG C und 80 DEG C, einer Brüdentemperatur von 15 DEG C bis 55 DEG C sowie einem Druck zwischen 0,5 und 1.000 mbar sowie einer Rührerumdrehungsgeschwindigkeit von 0 und 10 Upm und einer Zerhackerumdrehungsgeschwindigkeit zwischen 200 und 800 Upm getrocknet. Die gemäß der EP 0 753 306 eingesetzten Vakuumtrocknungssysteme werden beispielsweise von den früheren Firmen Inox Glatt AG oder Inox-Maurer AG im Handel unter den Bezeichnungen "IUT" oder "INOX" vertrieben. Heutige Hersteller und Vertreiber sind z.B. De Dietrich Process Systems GmbH, Mainz, Deutschland (Rosemund ®).

Bei diesem Vakuumtrocknungssystem wird im Batchverfahren der zu trocknende Fluidextrakt von oben in die Misch- und Trockenkammer eingepumpt und anschließend ein Vakuum angelegt.

Ein bevorzugtes Vakuumtrocknungssystem weist die folgenden Merkmale auf, wie z.B. in einer bekannten IUT/INOX Anlage (supra) verwirklicht:
a.) Ein sich durch eine zylindrische Misch- und Trocknerkammer erstreckendes mehrschenkliges Rührwerk mit eigenem Antrieb und je nach Erfordernissen Brüdenfilter, Rückspülungseinrichtung, Lösungsmittelkondensator mit Nachkühler und Auffanggefäß, Rückkondensator, eine Prozess-, Steuer- und Regeleinheit, sowie ggf. Granulierdüsen.
b.) Weiterhin kann ein sich über die Gesamttiefe der Trockner-und Mischkammer erstreckender Zerhacker mit rührwerksunabhängigem Antrieb vorgesehen sein sowie optional ein kammartiger Stator zur Erhöhung der Zerhackerwirkung.
c.) Des Weiteren kann optional zum Einbringen des flüssigen Pflanzenextraktes aus einem Reservoir in die Trocknerkammer eine oder mehrere Düsen vorgesehen sein, wie z.B. in WO2002073108 beschrieben.

Die derart erhaltenen Pflanzentrockenextrakte werden zu pharmazeutischen Zubereitungen weiter verarbeitet.

Das antimikrobiell wirkende Mittel, enthaltend den erfindungsgemäßen Pflanzentrockenextrakt, kann somit vorteilhaft bei der Behandlung von durch atemwegsrelevanten Erregern ausgelösten Infektionen eingesetzt werden. Die schleimlösende und entzündungshemmende Wirkung wird durch die zusätzliche antimikrobielle Wirkung ergänzt. Hierdurch wird eine Infektion der oberen Atemwege neben dem Lösen des zähflüssigen- mit Erregern beladenen Schleims abgeschwächt durch Abtöten und/oder Reduktion der Proliferation der bakteriellen Erreger eingedämmt oder sogar vollständig zum Erliegen gebracht.
Durch die oben beschriebene Erfindung wird ein z.B. an Sinusitis und / oder Rhinosinusitis und / oder Entzündungen der Nasennebenhöhlen, insbesondere in der jeweils akuten Form erkrankter Patient auf schonende Art und Weise ohne den Einsatz von synthetisch-chemischen Komponenten behandelt.

Die antimikrobiell wirkende (pharmazeutische) Zusammensetzung der vorliegenden Erfindung ist besonders gegen atemwegsrelevante Erreger wirksam, wobei sie speziell gegen grampositive Kokken wie Staphylococcus aureus, Staphylococcus aureus (MRSA), Staphylococcus epidermidis, Streptococcus pyogenes, Streptococcus pneumoniae und Streptococcus mutans, gegen gramnegative Stäbchenbakterien wie Haemophilus influenzae, sowie gegen Enterobacteriaceae faecalis antimikrobielle Wirksamkeit zeigt. Ferner wird gegen Viren eine Wirksamkeit erzielt.

Die galenische Formulierung des antimikrobiellen Mittels kann ausgewählt sein aus der Gruppe bestehend aus: Tropfen, Saft, Sirup, Tabletten, Dragees, Kapseln, Retard-Formulierungen, Rektal- oder Vaginalsuppositorien, Aufguss, insbesondere Rachenspray sowie Desinfektionslösungen; Salben, Emulsionen, Puder, Pulver, Nasenspray, flüssige oder feste Präparate für die Inhalation, Kompressen, Einlagen, insbesondere Wund- und Zahnfleischeinlagen, Tamponaden, auch für Zähne, Spüllösungen, insbesondere in Kombination mit physiologischen und hyperosmolaren Konzentrationen von Salzen oder Salzgemischen, bevorzugt Kochsalz, insbesondere Meersalz. Selbstverständlich kann die Formulierung pharmazeutisch übliche Hilfsstoffe enthalten.

Somit betrifft die vorliegende Erfindung auch die Verwendung oder Anwendung des erfindungsgemäßen antimikrobiellen Mittels enthaltend einen erfindungsgemäßen Pflanzentrockenextrakt zur Behandlung von durch atemwegsrelevanten Erregern ausgelösten Infektionen sowie die Verwendung zur Herstellung eines Arzneimittels als auch ein Arzneimittel als solches.

In einer weiteren Ausführungsform betrifft die Erfindung ein Arzneimittel zur Verwendung oder Anwendung bei Sinusitis und / oder Rhinosinusitis und / oder Entzündungen der Nasennebenhöhlen, insbesondere in der jeweils akuten Form, insbesondere zur Behandlung und Prophylaxe von Sinusitis und / oder Rhinosinusitis und / oder Entzündungen der Nasennebenhöhlen, insbesondere in der jeweils akuten Form.

Aufgrund der HNO-Relevanz der getesteten Erreger ist das erfindungsgemäße Mittel ebenfalls hervorragend überall dort geeignet, wo Bakterien direkt und unmittelbar lokal bzw. topisch bekämpft werden können.

Das erfindungsgemäße Mittel kann somit bevorzugt - neben der systemischen Applikation - unmittelbar am kontaminierten Ort, z.B. in Form eines biologisch zu 100% abbaubaren Desinfektionslösung oder natürlich auch zur direkten topischen Anwendung auf Haut und Schleimhäuten sowohl am Menschen als auch am Tier eingesetzt werden. Hierzu kommen unterschiedliche Formen der Anwendung in Betracht. Besonders geeignet sind die Formulierungen als Lösung, Cremes, Salben und Emulsionen im dermatologischen Bereich der Human- wie auch der Veterinärmedizin. Hier kann das erfindungsgemäße Mittel direkt auf die erkrankte Hautpartie appliziert werden und/oder in Form von getränkten Kompressen, Einlagen oder Tamponaden verwendet werden.

Ganz besonders interessant ist die Anwendung des erfindungsgemäßen Mittels natürlich im gesamten Bereich der Erkrankungen des gesamten Respirationstraktes, insbesondere der oberen Luftwege, hier vorzugsweise im Bereich der Rachen-, Nasen- und Nasennebenhöhlenschleimhäute. Besondere Bedeutung kommt der Nasenspülung, insbesondere in Verbindung mit Salzen, wie beispielsweise in Kombination mit einer physiologischen oder hyperosmolaren Salz-Lösung. Erfindungsgemäß umfasst ist ebenfalls ein Nasenspray enthaltend das erfindungsgemäße Mittel.

Von Tonsillenpinsellösungen, Gurgellösungen bei pharyngealen Infektionen bis hin zu Pulverinhaltionspräparaten oder Verneblerinhalationspräparaten reicht die breite Palette der neuen Anwendungsmöglichkeiten.

Weitere Anwendungs- und Indikationsbereiche umfassen Wund- und Zahnfleischeinlagen, z.B. in Form von Baumwolleinlagen oder Baumwollfadeneinlagen, welche mit dem erfindungsgemäßen Mittel getränkt sind.
Ebenso denkbar sind Ohrspülungen mit Lösungen, die das erfindungsgemäße Mittel enthalten, bei Gehörganginfektionen.

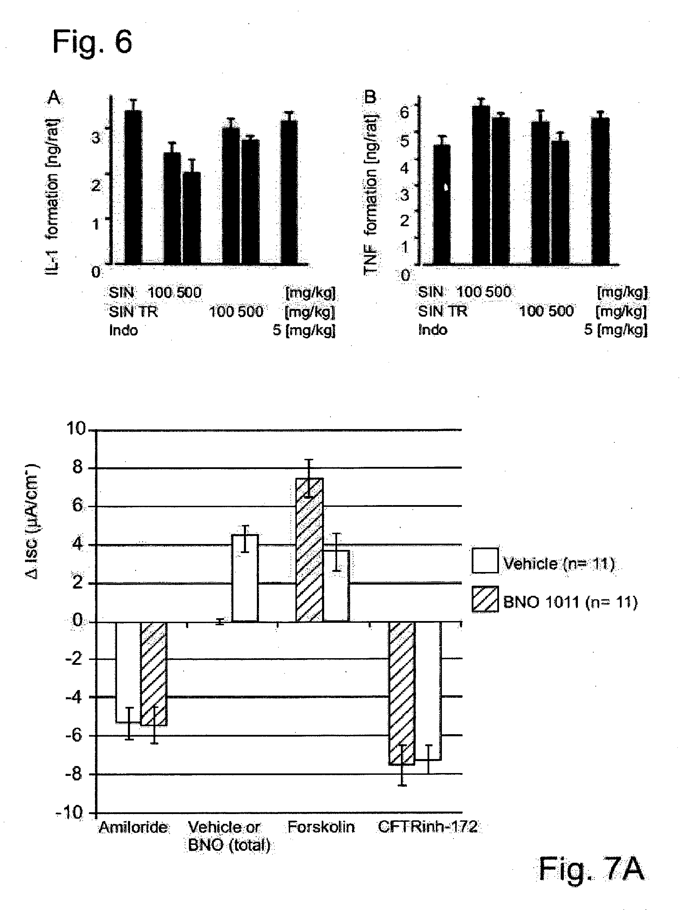

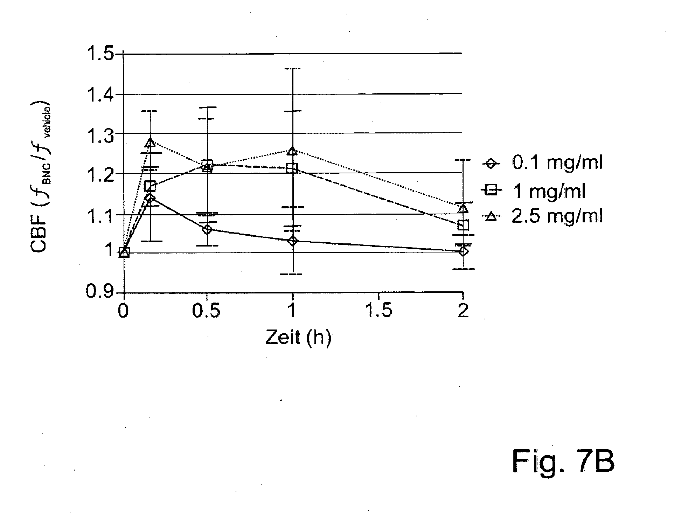

Daher betrifft die Erfindung ebenfalls ein Arzneimittel zur Verwendung und Anwendung von Erkrankungen des gesamten Respirationstraktes, insbesondere der oberen Luftwege, insbesondere im Bereich der Rachen-, Nasen- und Nasennebenhöhlenschleimhäute, Atemwegserkrankungen, insbesondere Mucovizidose (zystische Fibrose), insbesondere deren Behandlung und Prophylaxe. Besonders vorteilhaft kann eine Mucovizidose behandelt werden, vergleiche Figuren 7A und 7B.

Eine weitere bevorzugte Ausführungsform betrifft ein Nahrungsergänzungsmittel enthaltend das erfindungsgemäße Mittel, insbesondere in Form einer diätetischen Zusammensetzung. Geeignete erfindungsgemäße Nahrungs- oder Lebensmittel, einschließlich Wasser, sind solche wie z.B. in der Verordnung (EG) Nr. 178/2002 vom 28. Januar 2002 nicht abschließend definiert, solche wie Backwaren und Getränken und Kindernahrungszubereitungen. Das erfindungsgemäße Nahrungsergänzungsmittel kann mit einem geeigneten physiologisch verträglichen Träger versetzt werden.

Die erfindungsgemäßen pharmazeutischen Zubereitungen können in Form von Dosierungseinheiten hergestellt werden. Dies bedeutet, dass die Zubereitungen in Form einzelner Teile, vorzugsweise Kapseln und Ampullen vorliegen, deren Wirkstoffgehalt an Pflanzentrockenextrakten einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge des erfindungsgemäßen Pflanzentrockenextrakts (Wirkstoff), die bei einer Applikation verabreicht wird und die gewöhnlich einer ganzen, einer halben, einem Drittel oder einem Viertel einer Tagesdosis entspricht. Bevorzugt ist eine Dosierung von 3 - mal täglich, vorzugsweise in Form einer Tablette, insbesondere morgens, mittags und abends, ggfs. zu den Mahlzeiten.

In einer weiteren bevorzugten Ausführungsform kann die galenische Formulierung einer Calziummanteltablette gewählt werden, wie in EP EP1392337 offenbart.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen, wie a) Füll- und Streckmittel, z.B. Stärken, Milchzucker, Rohrzucker, Glukose, Mannit, Dextrine, Maltodextrin und Kieselsäure, hochdisperses Siliciumdioxid, b) Bindemittel, z.B. Carboxymethylcellulose, Cellulosepulver, mikrokristalline Cellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.
Die Tabletten, Dragees, Kapseln, Pillen und Granulate können mit den üblichen, gegebenenfalls Opakisierungsmittel enthaltenden Überzügen und Hüllen versehen sein, z.B. solche wie nicht abschließend Hypromellose, mikrokrisatlline Cellulose, Stearinsäure, Titandioxid, und ebenfalls so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

### Beispiele:

Diese Beispiele dienen ausschließlich zur Erläuterung der Erfindung, ohne die Erfindung auf diese Beispiele zu beschränken.

Nachstehend bedeutet "Trockenextrakt (TE)" der erfindungsgemäße hergestellte Pflanzentrockenextrakt.

### Beispiel 1:

Die anti-virale Aktivität des erfindungsgemäßen Trockenextrakts wurde in einer Vielzahl von in vitro - Versuchen bestätigt.

In diesen Versuchen wurde zunächst die allgemeine zellschädigende (zytotoxische) Wirkung des erfindungsgemäßen Trockenextrakts zum bekannten Sinupret® (alkoholisch/wässrig) untersucht. Nach Inkubation geeigneter Zelllinien (z.B. HeLa, HEp-2) mit unterschiedlichen Viren für die Dauer von einer Stunde, wurden die infizierten Zelllinien mit verschiedenen Konzentrationen behandelt, und anschließend die Wirkung auf die Virusvermehrung gemessen.

Die quantitative Bestimmung der antiviralen Aktivität in vitro erfolgte entweder über den Nachweis eines zytopathogenen Effekts (Adeno5 Virus), im Plaquereduktionsassay (FluA, HRV14, RSV) und in virusspezifischen Enzymimmunoassays (ELISA; Adeno5, RSV).

Beim Nachweis eines zytopathogenen Effekts werden die konfluent gewachsenen virussensitiven Zellen mit einer definierten Viruslösung (M.O.I., multiplicity of infection) infiziert. Nach einer einstündigen Inkubation wird das Virusinokkulum abgezogen und der infizierte Zellrasen gewaschen. Im Anschluss erfolgt die Zugabe der physiologischen Substanzkonzentrationen. Die jeweiligen Testansätze werden solange kultiviert bis in den unbehandelten Virus-Kontrollen mikroskopisch ein 70-90% -iger zytopathogener Effekt (CPE) zu beobachten ist, der sich als zerstörte Zellareale zeigt. Die Fläche der zerstörten Zellareale wird als 100% -ige Infektion definiert. Vergleichend werden die Zellflächen der jeweiligen Testansätze ausgewertet, so dass hemmende Effekte der zu analysierenden Substanzen als prozentuale Hemmung (% Hemmung) gezeigt werden können.

Beim Plaquereduktionsassay werden die konfluent gewachsenen virussensitiven Zellen mit einer definierten Viruslösung (M.O.I., multiplicity of infection) infiziert. Nach einer einstündigen Inkubation wird das Virusinokkulum abgezogen und der infizierte Zellrasen gewaschen. Im Anschluss erfolgt die Zugabe der physiologischen Substanzkonzentrationen sowie einer festen Mediumkomponente (Agarose oder Methylzellulose) und weitere Inkubation. Durch die feste Komponente im überlagerten Medium wird die Infektionsfläche begrenzt, so dass ein Herd von infizierten Zellen ("Plaque") entsteht. Die jeweiligen Testansätze werden solange kultiviert bis in den unbehandelten Virus-Kontrollen mikroskopisch die eingestellte Plaquezahl (M.O.I.) zu beobachten ist. Durch Fixierung und Färbung des Zellrasens können die Virusplaques als helle Höfe im dunkelgefärbten Zellrasen sichtbar gemacht werden. Die Bestimmung der Plaquezahl erfolgt mit Hilfe von Bildverarbeitungssystemen. Die Plaquezahl der unbehandelten Kontrolle wird als 100%ige Infektion definiert. Demgegenüber wird die Plaquezahl der jeweiligen Testansätze ausgewertet, so dass hemmende Effekte der Testsubstanz als prozentuale Hemmung (% Hemmung) gezeigt werden können.

Im ELISA wird die Virusproduktion analysiert. Teststreifen mit Antikörpern gegen die spezifischen Viren binden die im Zellkulturüberstand der infizierten Zelllinien befindlichen Viren. Um die Reaktion sichtbar zu machen, setzt man einen mit Peroxidase markierten erregerspezifischen Nachweisantikörper ein. Nach Zugabe eines Substrats/Chromogens sowie von Wasserstoffperoxid und Tetramethylbenzidin erfolgt eine Farbreaktion. Die Intensität der Färbung wird photometrisch bestimmt und ist dem Gehalt an Virusantigen proportional. Die Analyse der Virusproduktion bei infizierten und behandelten Zellen erfolgt nach Infektion konfluent gewachsener virussensitiver Zellen mit einer definierten Viruslösung (M.O.I., multiplicity of infection). Nach einer einstündigen Inkubation wird das Virusinokkulum abgezogen und der infizierte Zellrasen gewaschen. Im Anschluss erfolgt die Zugabe der physiologischen Substanzkonzentrationen. Die jeweiligen Testansätze werden solange kultiviert bis in den unbehandelten Virus-Kontrollen mikroskopisch ein 70-90% -iger zytopathogener Effekt (CPE) zu beobachten ist. Die neusynthetisierten Viren befinden sich in diesem Stadium im Zellkulturüberstand. Die photometrisch bestimmten Extinktionswerte der unbehandelten Kontrollen werden als 100%ige Infektion definiert. Vergleichend werden die Extinktionswerte der jeweiligen Testansätze ausgewertet, so dass hemmende Effekte der Testsubstanzen als prozentuale Hemmung (% Hemmung) dargestellt werden können.

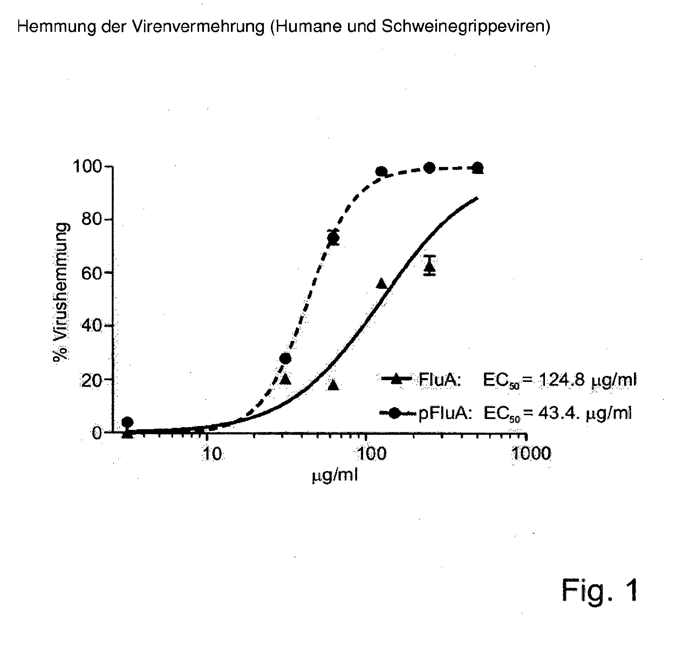

In allen Versuchen zeigte sich eine deutliche Hemmung der Virenvermehrung, d.h. eine Reduktion der Virenlast, siehe Figur 1.

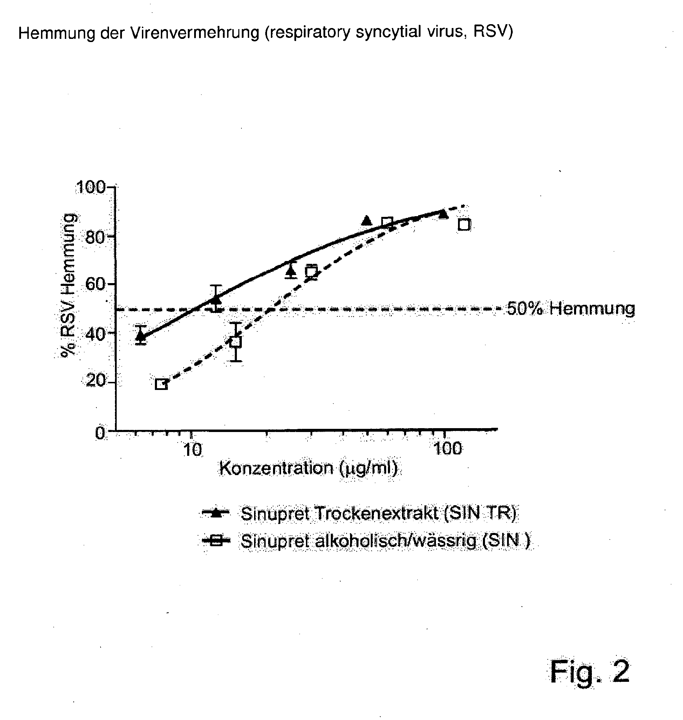

Der erfindungsgemäße Trockenextrakt inhibiert die Vermehrung von Humanen- (FluA) und Schweine- (pFluA) Influenzaviren (Grippeviren). Konzentrationen von 124.8 µg Sinupret/ml (FluA) bzw. 43.4 µg Sinupret/ml (pFluA) sind ausreichend um 50% der Viren(last) zu inhibieren (reduzieren). Der erfindungsgemäße Sinupret Trockenextrakt zeigt gegen die Virenstämme HRV 14, Adeno5 und RSV eine niedrigere EC50 und daher entsprechend eine höhere Wirksamkeit als Sinupret® alkoholisch/wässrig (siehe Figuren 1 und 2).

**Tabelle 0: Antivirale Wirkung**

| Virenstamm | Sinupret alkoholisch/wässrig EC50 [µg/ml] | Sinupret Trockenextrakt EC50 [µg/ml] |
|---|---|---|
| HRV 14 | 73,1 | 50,5 |
| Adeno 5 | 66, 4 | 13, 8 |
| RSV | 20,7 | 10, 4 |

### Beispiel 2:

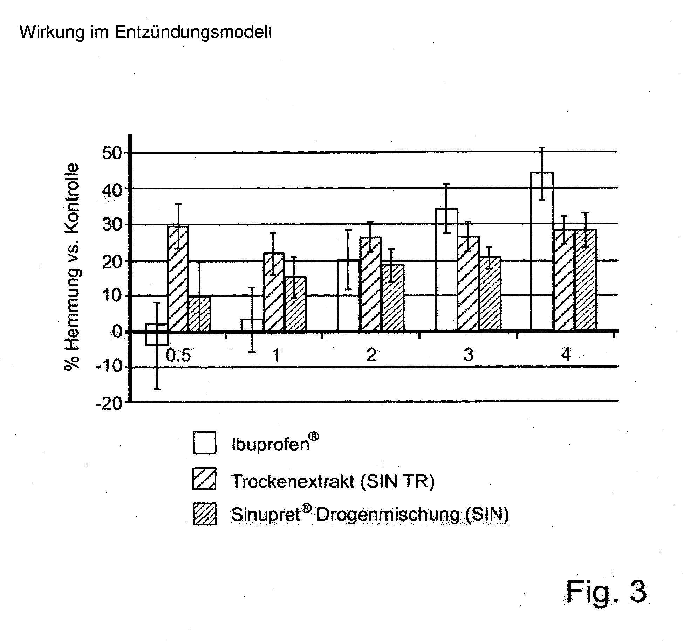

Die anti-entzündliche Aktivität von Sinupret konnte im Tiermodell bestätigt werden. Als Testmodell wurde z.B. das Carrageen-induzierte Pfotenödem bei der Ratte (Male Wistar Han rats, 220-230g) gewählt. In diesem Modell können Testsubstanzen auf ihre anti-entzündliche Wirkung untersucht werden, indem ihre hemmende Wirkung auf das durch Carrageen verursachte Pfotenödem oder Pleuritis gemessen wird. Als Referenzsubstanzen dienen (RS)-2-[4-(2-Methylpropyl) phenyl]propansäure (Ibuprofen®) und 2-[1-(4-Chlorbenzoyl)- 5-methoxy-2-methyl-1H-indol-3-yl] essigsäure (Indomethazin, "indo"). In den vorliegenden Versuchen wurden Gruppen von jeweils 10 Ratten entweder mit Ibuprofen®, Indomethazin, Sinupret® Trockenextrakt (SIN TR) oder Sinupret® Drogenmischung (SIN, wie käuflich erhältlich) behandelt und 1 Stunde später Carrageen injiziert. Die Hemmung der Ödembildung durch die Test- und Referenzsubstanzen wurde zu verschiedenen Zeitpunkten nach Carrageen-Injektion bestimmt, wobei die Pfotenvolumina von nur mit Carrageen behandelten Tieren als Kontrolle dienten (Vehicel = Blindkontrolle). Die Ergebnisse dieser Versuche sind in den Tabellen 1 und 2 und Figuren 3 bis 6 dargestellt und nachstehend erläutert.

Figur 3: Im Carrageen-Modell zeigte sich, dass der erfindungsgemäße Trockenextrakt das durch Carrageen induzierte Pfotenödem bereits 30 Minuten nach Carrageen-Injektion hemmt, und zwar stärker als die Sinupret Drogenmischung, während Ibuprofen® zu diesem frühen Zeitpunkt noch keine entzündungshemmende Wirkung zeigte. Auch ein und zwei Stunden nach Ödem-Induktion ist die anti-entzündliche Wirkung des erfindungsgemäßen Trockenextrakts noch stärker als diejenige von Ibuprofen® und Sinupret® Drogenmischung (SIN).

Tabelle 1 zeigt die Wirkung von Sinupret® (SIN) bei Carrageen induzierter Pleuritis anhand der Entzündungsmarker (PGE₂, LTB₄, TNF-alpha, IL1-beta) mit Messung nach 4h.

Ratten (jeweils 10 pro Gruppe) wurden mit jeweils 100 mg/kg oder 500 mg/kg SIN und vergleichend mit 5 mg/kg Indomethazin und Blindprobe behandelt und 1 Stunde später Carrageen injiziert.

"Inflammatory cells" korreliert mit PMN (polymorphonuclear neutrophils) Akkumulation / Infiltration.

Statistik: Mittel +/- SEM, n = 10, ** p< 0,01; ***p<0,001 vs vehicle (Blindprobe) (Tukey Test), p< 0,05 ist statistisch signifikant.

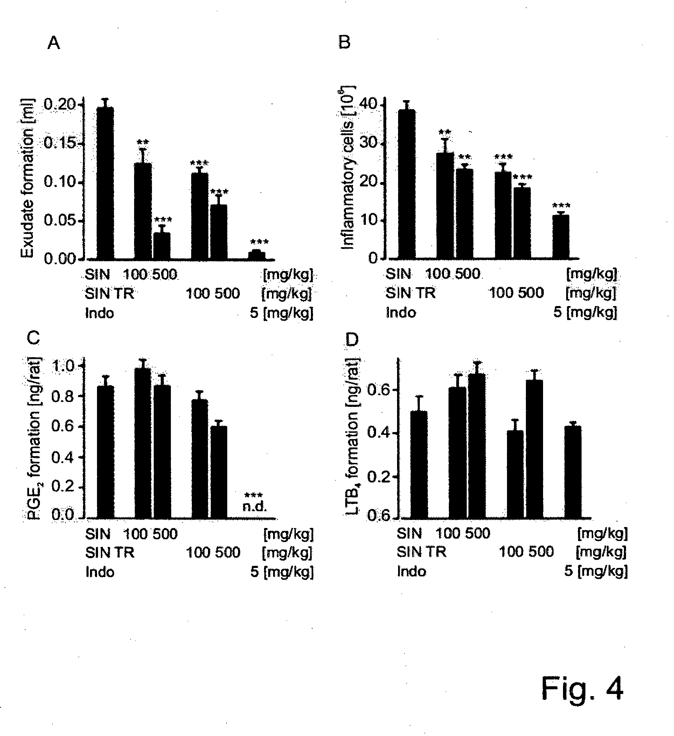

Eine vergleichende graphische Darstellung ist in Figur 4 gegeben.

| **treatment** | **exudate volume (ml)** | **inflammatory** | **PGE₂** | **LTB₄** | **TNFα** | **IL1β** |
|---|---|---|---|---|---|---|
| | | **cells × 10⁶** | **ng/rat** | **ng/rat** | **ng/rat** | **ng/rat** |
| **vehicle** | 0.196±0.012 | 38.6±2.54 | 0.861±0.061 | 0.50±0.07 | 4.94±0.33 | 3.37±0.25 |
| **Sinupret® 100 mg/kg** | 0.125±0.018 ** | 27.7±2.64 ** | 0.973±0.066 | 0.61±0.06 | 5.94±0.30 | 2.45±0.23 |
| | (36%) | (28%) | | | | (21%) |
| **Sinupret® 500 mg/kg** | 0.034±0.010 *** | 23.5±1.34 *** | 0.865±0.065 | 0.67±0.06 | 5.48±0.19 | 2.03±0.30 ** |
| | (83%) | (39%) | | | | (40%) |
| **indomethacin 5 mg/kg** | 0.009±0.003 *** | 11.4±0.93 *** | <0.125 *** | 0.43±0.02 | 5.48±0.28 | 3.17±0.18 |
| | (96%) | (70%) | | | | |

Tabelle 2 zeigt die Wirkung von Sinupret® Trockenextrakt (SIN TR) bei Carrageen induzierter Pleuritis anhand der Entzündungsmarker (PGE₂, LTB₄, TNF-alpha, IL1-beta) mit Messung nach 4h.

Ratten (jeweils 10 pro Gruppe) wurden mit jeweils 100 mg/kg oder 500 mg/kg SIN TR und vergleichend mit 5 mg/kg Indomethazin und Blindprobe behandelt und 1 Stunde später Carrageen injiziert.

"Inflammatory cells" korreliert mit PMN (polymorphonuclear neutrophils) Akkumulation / Infiltration.

Statistik: Mittel +/- SEM, n = 10, ** p< 0,01; ***p<0,001 vs vehicle (Blindprobe) (Tukey Test), p< 0,05 ist statistisch signifikant.

| **treatment** | **exudate volume** | **Inflammatory cells** | **PGE₂** | **LTB₄** | **TNFα** | **IL1β** |
|---|---|---|---|---|---|---|
| | **ml** | **x 10⁶** | **ng/rat** | **ng/rat** | **ng/rat** | **ng/rat** |
| **vehicle** | 0.192±0.012 | 38.6±2.54 | 0.891±0.061 | 0.50±0.07 | 4.94±0.33 | 3.37±0.21 |
| **dry extract 100 mg/kg** | 0.112±0.009 *** (43%) | 22.8±2.19 *** (41%) | 0.775±0.050 (10%) | 0.41±0.05 | 5.36±0.44 | 3.00±0.21 (11%) |
| **dry extract 500 mg/kg** | 0.072±0.013 *** (63%) | 18.8±0.92 (51%) | 0.603±0.039 ** (30%) | 0.64±0.05 | 4.66±0.30 | 2.74±0.09 (19%) |
| **indomethacin 5 mg/kg** | 0.009±0.003 *** (96%) | 11.4±0.93 *** (70%) | <0.125 *** | 0.43±0.02 | 5.48±0.28 | 3.17±0.18 |

Eine vergleichende graphische Darstellung ist in Figur 4 gegeben.

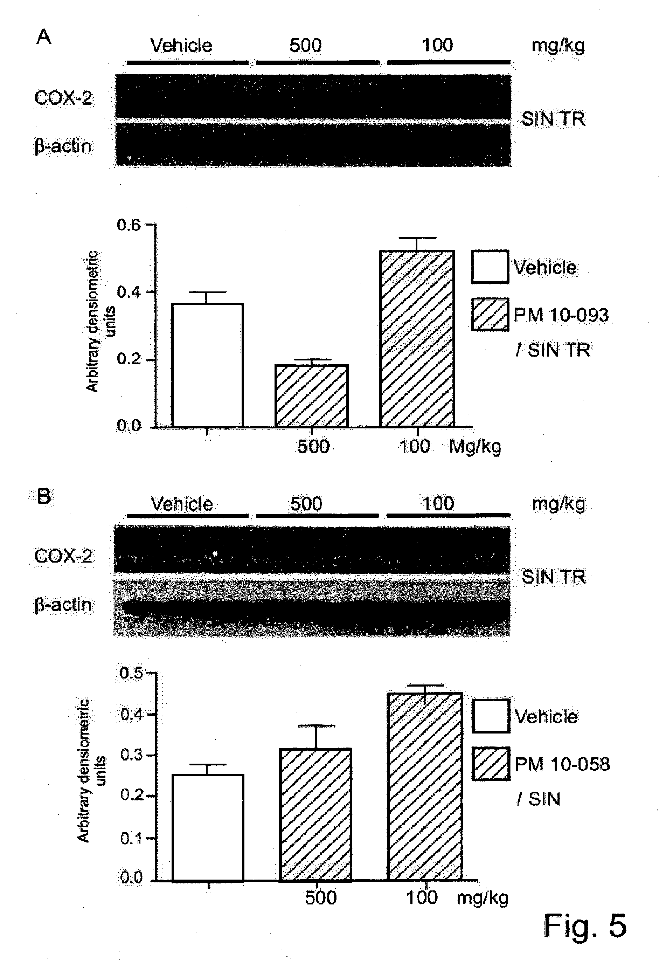

Figur 5 zeigt die Wirkung von Sinupret® Drogenmischung (SIN) und Sinupret Trockenextrakt (SIN TR) auf die Expression von COX-2 Protein in der Rattenlunge.

Ratten (jeweils 10 pro Gruppe) wurden mit jeweils 100 mg/kg oder 500 mg/kg SIN bzw. SIN TR und vergleichend mit 5 mg/kg Indomethazin und Blindprobe behandelt und 1 Stunde später Carrageen injiziert. Ein Western Blot wurde mit jeweils 30 µg Protein aus Rattenlunge (homogenisiert) auf 10 % SDS-Polyacrylamidgel durchgeführt und COX-2 analysiert.

Figur 6 zeigt die Wirkung von Sinupret® Drogenmischung (SIN) und Sinupret Trockenextrakt (SIN TR) auf Cytokine.

Ratten (jeweils 10 pro Gruppe) wurden mit jeweils 100 mg/kg oder 500 mg/kg SIN bzw. SIN TR und vergleichend mit 5 mg/kg Indomethazin und Blindprobe behandelt und 1 Stunde später Carrageen injiziert. Die Entzündungsmarker IL1-beta und TNF-alpha wurden 4h nach Carrageen-Injektion bestimmt.

Statistik: Mittel +/- SEM, n = 10, ** p< 0,01; ***p<0,001 vs vehicle (Blindprobe) (Tukey Test), p< 0,05 ist statistisch signifikant.

### Fazit:

Der erfindungsgemäße Sinupret Trockenextrakt (SIN TR) zeigt zumindest für PGE2 eine besonders vorteilhafte signifikante Inhibition der PGE2 Bildung (30%; p<0,01; Figur 4C, Tabelle 2) gegenüber Sinupret® Drogenmischung (SIN). Weiterhin ist der erfindungsgemäße Sinupret Trockenextrakt (SIN TR) bei niedriger Dosierung wirksamer als die bekannte Sinupret® Drogenmischung (SIN).

### Beispiel 3:

Im folgenden Beispiel wird gezeigt, dass der erfindungsgemäße Trockenextrakt bei topischer Verwendung die Chloridsekretion aktiviert, wahrscheinlich über die Aktivierung von CFTR. Zudem stimuliert der erfindungsgemäße Trockenextrakt die ziliare Schlagfrequenz.

Material: Zellkultur: Humane bronchiale epitheliale Zellen (HBE) wurden von Lonza (Walkersville, MD) erworben und mit bronchialen epithelialen Zellwachstumsmedium (BEGM) Lonza (Walkersville, MD) expandiert. 250 mg erfindungsgemäßer Trockenextrakt wurde in 1 ml zu 50 % Ethanol gelöst und im Ultraschall bei 35 kHz für 30 Minuten behandelt mit anschließender Zentrifugation bei 3000 g für 10 Minuten bei Zimmertemperatur. Der Überstand wurde abgesaugt. Amilorid (Sigma, St. Louis, MO) wurde im destillierten, deionisierten Wasser gelöst und 1000-fach verdünnt. Forskolin (Kaliokemm, EMD, San Diego, CA) wurde in DMSO gelöst und 1000-fach verdünnt.

Eine Ussing-Kammer (Physiology Instruments San Diego, CA, USA) wurde verwendet, enthaltend Transwell inserts (Corning Life Sciences) mit Durchführung bei 37 Grad und einem Monolayer mit einer Spannungsklemme von 0 Volt (VCC 600) (Physiology Cal Instruments San Diego, CA, USA) nach Einstellung des Flusswiderstandes. Transwellfilter wurden in der Lösung bei 37 Grad aufgestellt und die Lösung wurde kontinuierlich mit 95%-igen Sauerstoff zu 5%-igen CO₂ infundiert. Der transepitheliale Widerstand (RT) wurde mittels eines Computerprogramms (Physiology Instruments San Diego, CA, USA) ausgerichtet bei 640 ms, Bipolar 10 mV Potenzial über dem Monolayer nach dem Ohmschen Gesetz gemessen. Nach Definition ist ein positiver Ausschlag eine Anionsekretion oder Kationabsorpition. Die Experimente wurden zumindest 3-fach in HBE-Zellkulturen wiederholt.

Verwendete Elektrolytlösung (in mM): 120 NaCl, 25 NaHCO₃, 3,3 KH₂PO₄, 0,8 K₂HPO₄, 1,2 MgCl₂, 1,2 CaCl₂ und 10 Glukose.

Die cilialen Schlagfrequenzmessungen (CBF) wurden nach einer Methode gem. Woodworth et al (Woodworth, BA, Zhang S, Tamashiro E, Zinc increases ciliary beat frequency in a calcium dependent manner, Am J Rhinol Allergy 24: 6-10, 2010) durchgeführt. Die Bilder wurden mit Leica Microsystems, Inc., Bannockburn, IL mit einem 63-fachen Objektiv erstellt (Modell A 602f-2, High Speed Monogramme Digital Videokamera, Basler AG, Ahrensburg, Germany). Der erfindungsgemäße Trockenextrakt wurde auf den transepitheliale Elektrolyttransport untersucht. Der Trockenextrakt wurde in steigenden Konzentrationsmengen auf die basolaterale Oberfläche von HBE-Zellen in der Ussingkammer aufgebracht, bevor dieser anschließend mit Amilorid und Forskolin versetzt wurde.

In Figur 7a und Figur 7b zeigt der erfindungsgemäße Trockenextrakt nach Zugabe von Amilorid und Forskolin einen Wechsel des transepithelialen Kurzschluss-Stroms (I_{sc}) (7A), so dass eine dosisabhängige Anstieg der cilialen Schlagfrequenzmessungen (CBF) zu beobachten ist (7B), welches mit einer Chloridionensekretion einhergeht.

Diese Ergebnisse belegen den vorteilhaften Einsatz des erfindungsgemäßen Trockenextraktes z.B. mittels eines Nasensprays, da eine verbesserte mucociliary clearance (MCC) erreicht werden kann.

Fazit: Der erfindungsgemäße Trockenextrakt ist zur Behandlung von Atemwegserkrankungen, insbesondere zur Behandlung von Mucovizidose (zystische Fibrose) geeignet.

### Beispiel 4:

Die entzündungshemmende Wirkung des erfindungsgemäßen Sinupret Trockenextrakt und Sinupret Tropfen (alkolholisch/wässrig ("Sinupret OD")) wurde wie folgt untersucht.

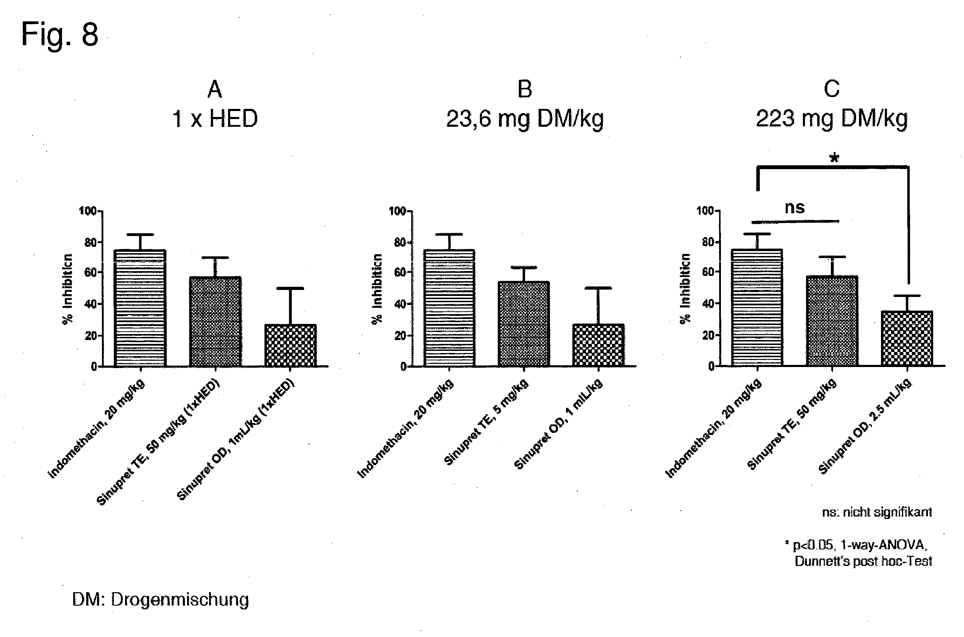

Carragen-induzierte Pfotenödem (supra): Zunächst wurden die Versuchstiere (n=8/Gruppe) im nüchternen Zustand gewogen und das Basalvolumen der Hinterpfote gemessen. Den Tieren wurden die Testsubstanzen schlundiert (10 mL/kg Körpermasse) verabreicht (Sinupret Trockenextrakt (TE): 5 mg/kg, äquivalent zur Menge an Drogenmischung in 1 mL Tropfen/kg; 50 mg TE/kg entsprechend der 1-fachen Humanäquivalenzdosis (1 x HED); Sinupret Tropfen (Sinupret OD): 1 mL OD/kg, entsprechend der 1-fachen Humanäquivalenzdosis (1 x HED); 2.5 mL OD/kg, äquivalent zur Menge an Drogenmischung in 50 mg TE/kg; Indomethacin: 20 mg/kg als Positivkontrolle; 10% v/v Ethanol als Vehikelkontrolle). Nach 60 min wurden die Tiere durch eine subkutane Injektion von Carrageen (0.1 mL einer 1 % w/v Lösung in physiologischer Kochsalzlösung) in die linke Hinterpfote (plantar) provoziert. Pfotenvolumina wurden mittels Plethysmographie vor (-1 h) und nach der Carrageen-Injektion (Studie 1: +1 h (Figur 8), Studie 2: +15 min, +30 min, +1h (Fiur 9)) bei allen Tieren bestimmt. Die prozentuale Inhibition der Pfotenschwellung vs. Vehikelkontrolle wurde für jedes Versuchstier individuell berechnet.

Die Ergebnisse sind vergleichend in Figur 8 gezeigt (Entzündungshemmung nach 1 Stunde).

Figur 8: A: Sinupret TE und Sinupret OD wurden jeweils in der 1fachen Humanäquivalenzdosis verabreicht. Sinupret TE wirkt schneller und stärker entzündungshemmend als Sinupret OD. Die Entzündungshemmung durch Sinupret TE, nicht durch Sinupret OD, ist nach 1h vergleichbar mit der Hemmwirkung durch das bekannte Antiphlogistikum Indomethacin.

B, C: Die hier verabreichte Dosis von Sinupret TE und Sinupret OD ist auf Basis der jeweils zur Herstellung eingesetzten Menge an Drogenmischung (DM) (B: 23,6 mg/kg, C: 223 mg/kg) vergleichbar. Sinupret TE wirkt schneller und stärker entzündungshemmend als Sinupret OD. Die Entzündungshemmung durch Sinupret TE, nicht durch Sinupret OD, ist nach 1h vergleichbar mit der Hemmwirkung durch das bekannte Antiphlogistikum Indomethacin.

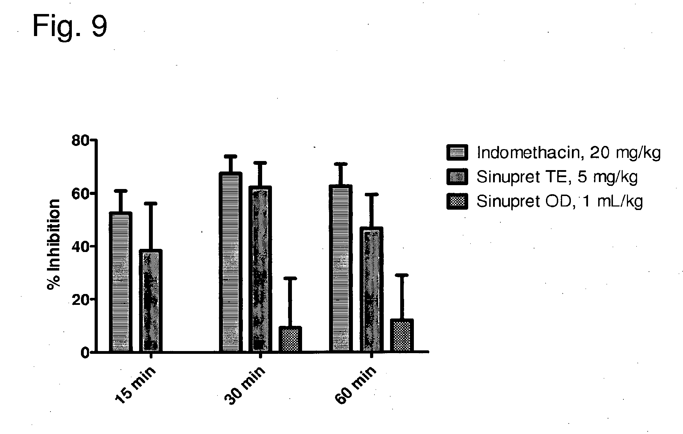

Die Ergebnisse sind vergleichend in Figur 9 gezeigt (Entzündungshemmung nach +15 min, +30 min, +1 h)

Figur 9: Die verabreichte Dosis von Sinupret TE und Sinupret OD ist auf Basis der jeweils zur Herstellung eingesetzten Menge an Drogenmischung (23,6 mg Drogenmischung/kg) vergleichbar. Sinupret TE zeichnet sich durch einen früheren und stärkeren Wirkeintritt im Vergleich zu Sinupret OD aus. Sinupret OD bewirkt erst ab 30 min eine Entzündungshemmung. Sinupret TE hemmt die Entzündung im kompletten untersuchten Zeitraum vergleichbar stark und schnell wie das bekannte Antiphlogistikum Indomethacin.

### Beispiel 5:

Das folgende Beispiel beschreibt bestimmte Markerverbindungen aus dem Vielstoffgemisch.

### Durchführung:

Sämtliche Proben wurden unmittelbar nach den einzelnen Extraktionsschritten entnommen, filtriert und in einer Konzentration von 600 mg/L (in 30 %-Vol. MeOH) massenspektrometrisch analysiert. Als interner Standard wurde Methylparaben verwendet. Die Proben wurden zweifach aufgearbeitet und zweifach analysiert. Es wurden folgende Parameter und Geräte verwendet:
MS: 5600 Triple ToF (ABSciex); HPLC: Agilent 1290
Software: Analyst TF 1.5.1, MultiQuant 2.1.1, MarkerView 1.2.1
Stationäre Phase: Zorbax RRHD Eclipse Plus C18, 2.1 x 50 mm, 1.8 µm

### LC-Methode:

| Schritt | Zeit (Min.) | Flussrate (µL/Min) | Laufmittel A | Laufmittel B |
|---|---|---|---|---|
| 0 | 0,00 | 600 | 95,0 | 5,0 |
| 1 | 1,00 | 600 | 95,0 | 5,0 |
| 2 | 6,00 | 600 | 69,0 | 31,0 |
| 3 | 10,00 | 600 | 0,0 | 100,0 |
| 4 | 11,00 | 600 | 0,0 | 100,0 |
| 5 | 11,10 | 600 | 95,0 | 5,0 |
| 6 | 13,00 | 600 | 95,0 | 5,0 |

| | | | | |
|---|---|---|---|---|
| A = 0,1 % Ameisensäure in H2O; B = Acetonitril | | | | |

### MS-Methode:

| Scan Type: | negative TOF MS |
|---|---|
| Duration | 10,997 mins |
| Cycle Time | 0,2750 secs |
| GS1 (Spray Gas): | 70,00 |
| GS2 (Turbo Gas): | 55,00 |
| CUR (Curtain Gas): | 25,00 |
| TEM (GS 2): | 500.0 |
| ISVF (Ion Spray Voltage Floating): | 4500,0 |
| CAD (Collision Gas): | 6 |
| TOF Masses (Da): | 130 - 2000 |
| Accumulation Time (sec): | 0,25 |
| Time Bins to sum: | 4 |
| DP: | -100,0 |
| CE: | -10,0 |

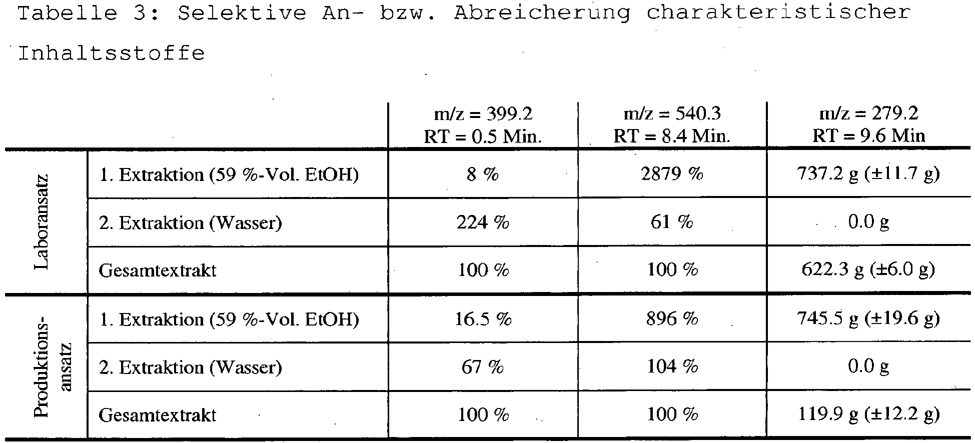

Sofern über Referenzstandards quantifiziert, werden die Inhaltsstoffmengen als absoluter Gehalt in [g] angegeben. Ansonsten wird der Inhaltsstoffgehalt in Relation [%] auf den jeweiligen Gesamtextrakt (nach Schritt f.)) angegeben. Die Daten entstammen einem Laboransatz A1 sowie einem Produktionsansatz P1. Die Signale wurden bei negativer Ionisierung detektiert.

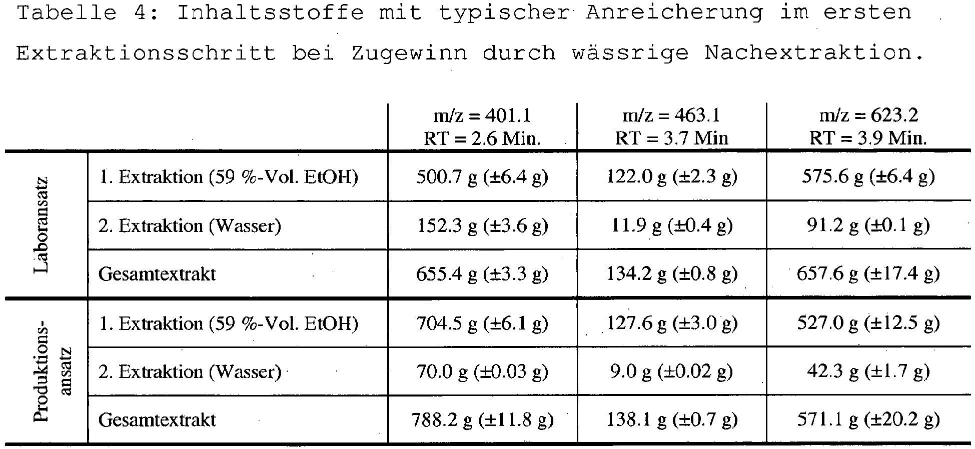

Sofern über Referenzstandards quantifiziert, werden die Inhaltsstoffmengen als absoluter Gehalt in [g] angegeben. Ansonsten wird der Inhaltsstoffgehalt in Relation [%] auf den jeweiligen Gesamtextrakt (nach Schritt f.)) angegeben. Die Daten entstammen dem Laboransatz A1 sowie dem Produktionsansatz P1. Die Signale wurden bei negativer Ionisierung detektiert.

## Patentansprüche

1. Verfahren zur Herstellung von Pflanzentrockenextrakten, mit den Schritten:
a.) alkoholische/wässrige Extraktion von Rumicis herba, Verbena officinalis, Sambucus nigra, Primula veris und Gentiana lutea,
b.) Abtrennen des Überstandes,
c.) wässrige Extraktion des Rückstandes,
d.) Abtrennen des Überstandes,
e.) Vereinen der erhaltenen Überstände aus b.) und d.),
f.) Trocknen der Überstände und Erhalten des Pflanzentrockenextraktes.

2. Verfahren nach Anspruch 1, wobei das Verhältnis von Gentiana lutea : Rumicis herba : Verbena officinalis : Sambucus nigra : Primula veris 1:3:3:3:3, jeweils +/- 0,3 bis 0,5 in Schritt a.) beträgt.

3. Verfahren nach Anspruch 1 oder 2, wobei im Schritt a.) das wässrige/alkoholische Extraktionsmittel 40 : 60 (v/v) bis 60 : 40 (v/v), insbesondere 41 : 59 (v/v), 50 : 50 (v/v) beträgt.

4. Verfahren nach einem der vorhergehenden Ansprüche, **dadurch gekennzeichnet, dass** als Alkohol Ethanol, insbesondere 96%-iger Ethanol verwendet wird.

5. Verfahren nach einem der vorhergehenden Ansprüche, **dadurch gekennzeichnet, dass** die Pflanzen in a.) gemeinsam in einem Batch vorgelegt werden.

6. Verfahren nach einem der vorhergehenden Ansprüche, **dadurch gekennzeichnet, dass** die Extraktion in den Schritten a.) und c.) bei 20 bis 40 DEG C durchgeführt wird.

7. Verfahren nach einem der vorhergehenden Ansprüche, **dadurch gekennzeichnet, dass** die Extraktion in den Schritten a.) und c.) in 2 bis 8h durchgeführt wird.

8. Verfahren nach einem der vorhergehenden Ansprüche, **dadurch gekennzeichnet, dass** das Trocknen gemäß Schritt f.) im Vakuum bei 30 bis 60 DEG C, insbesondere bei 40 bis 50 DEG C erfolgt.

9. Verfahren nach einem der vorhergehenden Ansprüche, **dadurch gekennzeichnet, dass** das Trocknen gemäß Schritt f.) in einem Vakuumrührwerktrockner erfolgt.

10. Pflanzentrockenextrakte erhältlich nach einem der Ansprüche 1 bis 9.

11. Pharmazeutische Zubereitungen enthaltend Pflanzentrockenextrakte nach Anspruch 10, ggfs. samt geeigneter Trägerstoffe, insbesondere in Form von Dragees, Tabletten, Filmtabletten, Pulver, Kapseln oder flüssige Verdünnungen, insbesondere Tropfen, Säfte oder Sirupe, Salben, Emulsionen, Puder, Pulver, Nasenspray, flüssige oder feste Präparate für die Inhalation, Kompressen, Wund- und Zahnfleischeinlagen, Tamponaden, Tonsillenpinsellösungen, Gurgellösungen oder Spüllösungen.

12. Arzneimittel enthaltend ein Pflanzentrockenextrakt nach Anspruch 10 oder eine pharmazeutische Zubereitung nach Anspruch 11.

13. Antibakterielles, antivirales oder anti-entzündliches Mittel enthaltend ein Pflanzentrockenextrakt nach Anspruch 10 oder eine pharmazeutische Zubereitung nach Anspruch 11.

14. Arzneimittel enthaltend ein Pflanzentrockenextrakt nach einem der Ansprüche 10 bis 12 zur Verwendung in der Behandlung und Prophylaxe von Sinusitis und / oder Rhinosinusitis und / oder Entzündungen der Nasennebenhöhlen, insbesondere in der jeweils akuten Form.

15. Arzneimittel enthaltend ein Pflanzentrockenextrakt nach einem der Ansprüche 10 bis 12 zur Verwendung in der Behandlung und Prophylaxe von Erkrankungen des gesamten Respirationstraktes, insbesondere der oberen Luftwege, insbesondere im Bereich der Rachen-, Nasen- und Nasennebenhöhlenschleimhäute, Atemwegserkrankungen, insbesondere Mucovizidose (zystische Fibrose).

16. Nahrungsergänzungsmittel enthaltend ein Pflanzentrockenextrakt nach Anspruch 10.

## Claims

1. A method for producing dry plant extracts, said method comprising the following steps:
a.) alcoholic/aqueous extraction of Rumicis herba, Verbena officinalis, Sambucus nigra, Primula veris and Gentiana lutea,
b.) separation of the supernatant,
c.) aqueous extraction of the residue,
d.) separation of the supernatant,
e.) combining of the obtained supernatants from b.) and d.),
f.) drying of the supernatants and provision of the dry plant extract.

2. The method according to Claim 1, wherein the ratio in step a.) of Gentiana lutea : Rumicis herba : Verbena officinalis : Sambucus nigra: Primula veris is 1:3:3:3:3, in each case +/- 0.3 to 0.5.

3. The method according to Claim 1 or 2, wherein, in step a.), the aqueous/alcoholic extraction agent is 40 : 60 (v/v) to 60 : 40 (v/v), in particular 41 : 59 (v/v), or 50 : 50 (v/v).

4. The method according to one of the preceding claims, **characterized in that** ethanol, in particular 96 % ethanol, is used as alcohol.

5. The method according to one of the preceding claims, **characterized in that** the plants in a.) are provided together in a batch.

6. The method according to one of the preceding claims, **characterized in that** the extraction in steps a.) and c.) is carried out at 20 to 40 °C.

7. The method according to one of the preceding claims, **characterized in that** the extraction in steps a.) and c.) is carried out in 2 to 8 h.

8. The method according to one of the preceding claims, **characterized in that** the drying process according to step f.) is carried out under vacuum at 30 to 60 °C, in particular at 40 to 50 °C.

9. The method according to one of the preceding claims, **characterized in that** the drying process according to step f.) is carried out in a vacuum agitated dryer.

10. Dry plant extracts obtainable in accordance with one of Claims 1 to 9.

11. Pharmaceutical preparations comprising dry plant extracts according to Claim 10, where applicable including suitable carrier substances, in particular in the form of dragées, tablets, film-coated tablets, powder, capsules or liquid dilutions, in particular drops, juices or syrups, ointments, emulsions, granulates, powders, nasal sprays, liquid or solid preparations for inhalation, compresses, wound and gum dressings, tamponades, tonsil paint solutions, gargling solutions or rinsing solutions.

12. A pharmaceutical comprising a dry plant extract according to Claim 10 or a pharmaceutical preparation according to Claim 11.

13. An antibacterial, antiviral or anti-inflammatory agent comprising a dry plant extract according to Claim 10 or a pharmaceutical preparation according to Claim 11.

14. A pharmaceutical comprising a dry plant extract according to one of Claims 10 to 12 for use in the treatment and prophylaxis of sinusitis and/or rhinosinusitis and/or inflammation of the nasal sinuses, in particular in acute form in each case.

15. A pharmaceutical containing a dry plant extract according to one of Claims 10 to 12 for use in the treatment and prophylaxis of diseases of the entire respiratory tract, in particular of the upper airways, in particular in the region of the throat, nose and nasal sinus mucous membranes, and respiratory diseases, in particular mucoviscidosis (cystic fibrosis).

16. A dietary supplement containing a dry plant extract according to Claim 10.

## Revendications

1. Procédé de fabrication d'extraits secs de plantes, avec les étapes suivantes :
a.) extraction alcoolique/aqueuse de Rumicis herba, Verbena officinalis, Sambucus nigra, Primula veris et de Gentiana lutea,
b.) séparation du produit obtenu,
c.) extraction aqueuse du résidu,
d.) séparation du produit obtenu,
e.) réunion des produits obtenus en b.) et en d.),
f.) séchage des produits obtenus et obtention de l'extrait sec de plantes.

2. Procédé selon la revendication 1, dans lequel les rapports respectifs Gentiana lutea : Rumicis herba : Verbena officinalis : Sambucus nigra : Primula veris sont égaux à 1 : 3 : 3 : 3 : 3, +/- 0,3 à 0,5 dans l'étape a.).

3. Procédé selon les revendications 1 ou 2, dans lequel, dans l'étape a.), le produit d'extraction aqueux/alcoolique est dans un rapport de 40 : 60 (v/v) à 60 : 40 (v/v), notamment, de 41 : 59 (v/v), 50 : 50 (v/v).

4. Procédé selon l'une des revendications précédentes, **caractérisé en ce qu'**en tant qu'alcool, on utilise de l'éthanol, notamment de l'éthanol à 96 %.

5. Procédé selon l'une des revendications précédentes, **caractérisé en ce que** les plantes sont présentes dans un lot d'ensemble dans l'étape a.).

6. Procédé selon l'une des revendications précédentes, **caractérisé en ce que** l'extraction dans les étapes a.) et c.) est réalisée entre 20 et 40 degrés C.

7. Procédé selon l'une des revendications précédentes, **caractérisé en ce que** l'extraction dans les étapes a.) et c.) est réalisée pendant 2 à 8 heures.

8. Procédé selon l'une des revendications précédentes, **caractérisé en ce que** le séchage selon l'étape f.) est effectué sous vide entre 30 et 60 degrés C, notamment entre 40 et 50 degrés C.

9. Procédé selon l'une des revendications précédentes, **caractérisé en ce que** le séchage selon l'étape f.) est effectué dans un sécheur agitateur sous vide.

10. Extrait sec de plantes pouvant être obtenu selon l'une des revendications 1 à 9.

11. Préparations pharmaceutiques contenant des extraits secs de plantes selon la revendication 10, éventuellement avec des produits supports appropriés, notamment sous forme de dragées, de comprimés, de comprimés pelliculés, de produits pulvérulents, de capsules ou de dilutions liquides, en particulier, de gouttes, de jus ou de sirops, de pommades, d'émulsions, de poudres, de produits pulvérulents, de spray nasal, de préparations liquides ou solides pour l'inhalation, de compresses, de pansements pour les plaies et les gencives, de tamponnades, de solutions de badigeonnage des amygdales, de solutions pour gargariser ou de solutions de rinçage.

12. Produit pharmaceutique contenant un extrait sec de plantes selon la revendication 10 ou une préparation pharmaceutique selon la revendication 11.

13. Produit antibactérien, antiviral ou anti-infectieux contenant un extrait sec de plantes selon la revendication 10 ou une préparation pharmaceutique selon la revendication 11.

14. Produit pharmaceutique contenant un extrait sec de plantes selon l'une des revendications 10 à 12, pour une utilisation dans le traitement et la prophylaxie de la sinusite et/ou de la rhino sinusite, et/ou d'infections des voies sinusoïdales, notamment sous la forme respectivement aigüe.

15. Produit pharmaceutique contenant un extrait sec de plantes selon l'une des revendications 10 à 12, pour une utilisation dans le traitement et la prophylaxie de maladies du tractus respiratoire dans son ensemble, notamment des voies aériennes supérieures, en particulier, dans les parties du pharynx, du nez et des muqueuses des voies nasales, des maladies respiratoires, notamment de la mucoviscidose (fibrose kystique).

16. Complément alimentaire contenant un extrait sec de plantes selon la revendication 10.
